# EUROPEAN PATENT SPECIFICATION

(11) **EP 3 162 435 B1**
(45) Date of publication and mention of the grant of the patent: **11.03.2020**
(21) Application number: 15812185.5
(22) Date of filing: 25.06.2015
(51) Int. Cl.: B01J 19/08, B01J 19/12, C01C 1/04, A61L 2/10, A61L 2/14, C07C 221/00, C07C 223/06, C01B 13/02, C01B 13/11, C01B 3/04

(54) **METHOD FOR MANUFACTURING REACTION PRODUCT IN WHICH PHASE INTERFACE REACTION IS EMPLOYED AND PHASE INTERFACE REACTOR**
VERFAHREN ZUR HERSTELLUNG EINES REAKTIONSPRODUKTES MIT PHASENSCHNITTSTELLENREAKTION UND PHASENSCHNITTSTELLENREAKTOR
PROCÉDÉ DE PRODUCTION DE PRODUIT DE RÉACTION DANS LEQUEL UNE RÉACTION D'INTERFACE DE PHASE EST UTILISÉE ET RÉACTEUR D'INTERFACE DE PHASE

(30) Priority: 27.06.2014 JP 2014132690
(43) Date of publication of application: 03.05.2017
(73) Proprietor: Kyushu Institute of Technology, Kitakyushu-shi, Fukuoka 804-8550 (JP); Ebara Jitsugyo Co. Ltd., Chuo-ku, Tokyo 104-8174 (JP)
(72) Inventor: HARUYAMA, Tetsuya, Kitakyushu-shi Fukuoka 808-0196 (JP)
(74) Representative: Schäfer, Matthias W.
(86) International application number: PCT/JP2015/003207
(87) International publication number: WO 2015/198608

(56) References cited:
- WO-A2-2005/005009
- JP-A- 2003 159 570
- JP-A- 2008 090 919
- JP-A- 2010 166 855
- JP-A- 2010 194 379
- JP-A- 2012 521 240
- JP-A- 2014 012 626
- US-A1- 2012 093 691
- US-A1- 2012 181 168
- US-B1- 6 872 365

## Description

### Technical Field

The present invention relates to: a method for producing a reaction product using a phases interface reaction occurring at a phases interface between a plasma phase and a liquid phase in contact with the plasma phase; and a phases interface reactor.

### Background Art

Passing oxygen molecules through a discharge space causes conversion of oxygen to plasma and hence production of ozone, and irradiating this ozone with ultraviolet light induces production of hydroxy radical etc. Water purification systems and the like making use of this principle and high oxidizing power of the hydroxy radical etc. have been developed (see Patent Literatures 1 and 2). The production of hydroxy radical from ozone takes place according to the following reaction formulae (1) and (2).

O₃ + hv (UV) → ³O₂ + O (1)

O + H₂O + hv (UV) → 2HO· (2)

In the water purification systems mentioned above, water molecules (water vapor) contained in air react with oxygen atoms to form hydroxy radicals (the above formula (2)). This cannot generate a large amount of hydroxy radicals since the content of water vapor in air is at most approximately 3 to 4 vol%. It may be possible to generate ozone (plasma) under high humidity conditions; however, a high humidity hinders efficient discharge, leading to a failure to generate a sufficient amount of ozone.

Ammonia is important as a material for production of nitrogen fertilizer, urea etc., and a well-known method for industrial production of ammonia is the Haber-Bosch process. In this process, nitrogen and hydrogen are reacted at high temperature and pressure with the help of an iron-based catalyst to synthesize ammonia. Hydrogen, which is one of the materials for ammonia synthesis, is obtained usually by water vapor modification of underground hydrocarbons typified by petroleum, coal, natural gas, and the like. For such reasons, the Haber-Bosch process is an extensive reaction process that requires a huge amount of energy, as well as being an environmentally unfriendly reaction process that involves production of carbon dioxide as a by-product.

To overcome such disadvantages of the Haber-Bosch process, a method employing water-splitting reaction to obtain hydrogen has been proposed (see Patent Literature 3). This proposed method makes use of solar thermal energy to heat a heat medium and makes use of the thermal energy of the heat medium to cause water-splitting reaction, thus obtaining hydrogen. This method, which makes use of natural energy, has the merit of reducing environmental load and alleviating the burden of collecting high solar thermal energy.

The method as described above which employs water-splitting reaction to obtain hydrogen is an environmentally friendly method that produces no carbon dioxide, and is more advantageous than the Haber-Bosch process. Still, the method requires not only a large amount of energy to decompose water and obtain hydrogen but also a large amount of energy to react the obtained hydrogen with nitrogen, and further requires a catalyst for ammonia synthesis. An improvement in terms of cost is thus desired. US2012/0093691 discloses an air decontamination device and method wherein the device comprises a non-thermal plasma cell, an ultraviolet emitting device and an ozone depletion catalyst.

### Citation List

### Patent Literature

Patent Literature 1 Japanese Patent Laid-Open No. 2013-158706
Patent Literature 2 Japanese Patent Laid-Open No. 2013-154145
Patent Literature 3 Japanese Patent Laid-Open No. 2013-241303

### Summary of Invention

### Technical Problem

The present invention has been made in view of the above circumstances, and has as its object to provide: a method for producing a reaction product using a phases interface reaction that allows a plasmatic substance nitrogen plasma) to react efficiently with water or the like; and a phases interface reactor used for the method. In addition, a method for producing a secondary reaction product using the reaction product is described. Solution to Problem

A method for producing a reaction product including ammonia using a phases interface reaction according to the first invention adapted for the above object comprises: a plasma supply step of supplying a plasmatic substance into a reaction chamber, the plasmatic substance being produced by supplying nitrogen gas into a plasma generator; a water/aqueous solution supply step of supplying water or an aqueous solution into the reaction chamber; and an ultraviolet irradiation step of irradiating the plasmatic substance in the reaction chamber with ultraviolet light, wherein the humidity in the reaction chamber (11) is 40% or more and 70% or less during the ultraviolet irradiation step, the plasmatic substance and the water or a solute contained in the aqueous solution being reacted at a phases interface in the reaction chamber to produce the reaction product.

In the method for producing a reaction product using a phases interface reaction according to the first invention, a plasmatic substance and water or a solute in an aqueous solution are reacted at a phases interface between a plasma phase and a liquid phase in contact with the plasma phase. This provides a large area of contact between the two components, thus allowing the reaction to take place efficiently. In this method, a reaction occurs between the plasmatic substance and gaseous water (water vapor). The plasma can be generated in a place separate from the reaction site (the interior of the reaction chamber) having a certain humidity, and then supplied to the reaction site. This prevents a decrease in the efficiency of plasma generation. The term "plasma" as used herein refers, regardless of how the plasma is produced, to a gas or gaseous substance consisting of a particle ensemble including positively charged particles and negatively charged electrons that are distributed to substantially maintain electroneutrality or an ensemble including the particle ensemble and another atomic or molecular gas. Thus, the term "plasmatic" as used herein means that a part or all of the molecules constituting a gas are in an ionized or dissociated state, that atoms resulting from dissociation are in an associated state, or that such molecules and atoms are present together. Specifically, the ionized state of a substance is a state consisting of ions of the substance and electrons, the dissociated state of a substance is a state where the substance is present in the form of, for example, nitrogen atoms, and the associated state of atoms of a substance is a state where the substance is present in the form of, for example, ozone (O₃). For example, plasmatic oxygen (oxygen plasma) typically consists of a mixture of ozone (O₃), oxygen molecules (O₂), oxygen atoms (O), etc. Plasmatic nitrogen (nitrogen plasma) consists largely of nitrogen atoms (N). The "plasmatic substance" can be a gaseous ultraviolet-sensitive substance such as nitrogen atoms.

It is preferable that the method for producing a reaction product using a phases interface reaction according to the first invention comprise: a plasma supply step of supplying a plasmatic substance into a reaction chamber; a nebulization step of generating a mist of water or a mist of aqueous solution in the reaction chamber; and an ultraviolet irradiation step of irradiating the plasmatic substance in the reaction chamber with ultraviolet light, with the humidity (relative humidity) in the reaction chamber being 40% or more and 70% or less and that the plasmatic substance and the mist of water or a solute contained in the mist of aqueous solution be reacted at a phases interface in the reaction chamber. Since the humidity in the reaction chamber is less than 100%, it is possible, for example, to prevent ultraviolet light from being predominantly absorbed by water molecules, thus allowing the reaction to take place efficiently. In addition, the reaction product can be collected efficiently in the form of a gas because the humidity is less than 100%. If the humidity is 100%, a large amount of dew drops (water droplets) are formed on the inner surface of the reaction chamber, and an increased amount of water-soluble reaction product is dissolved in the water droplets, which imposes the need for collecting both the gas and liquid.

In the method for producing a reaction product using a phases interface reaction according to the first invention, the humidity in the reaction chamber is 40% or more and 70% or less during the ultraviolet irradiation step. When the humidity is in the above range, the reaction efficiency can be enhanced.

In the method for producing a reaction product using a phases interface reaction according to the first invention, it is preferable that the nebulization step be accomplished by heating the water or the aqueous solution in the reaction chamber. When the mist is generated by heating, the humidity or the size of mist particles can easily be well controlled. In addition, nebulization of water or the like in the reaction chamber allows all of the generated mist and water vapor to be irradiated with ultraviolet light, thus leading to high efficiency. Furthermore, heating in the reaction chamber results in an increase in the temperature in the reaction chamber and thus an increase in the amount of saturated water vapor. That is, the amount of water vapor present in the reaction chamber can be increased, and thus the reaction efficiency can be enhanced.

In the method for producing a reaction product using a phases interface reaction according to the first invention, the substance comprises, nitrogen. The use of nitrogen plasma allows synthesis of ammonia. Furthermore, this ammonia can be decomposed to produce hydrogen (H₂).

In the method for producing a reaction product using a phases interface reaction according to the first invention, it is preferable that the method further comprise a decomposition reaction step of decomposing ammonia produced by the reaction at the phases interface. This makes it possible to obtain hydrogen molecules.

A phases interface reactor according to the invention adapted for the above object comprises: a reaction chamber; plasma supply means configured for supplying a plasmatic substance into the reaction chamber; water/aqueous solution supply means configured for supplying water or an aqueous solution into the reaction chamber; and ultraviolet irradiation means configured for irradiating the plasmatic substance in the reaction chamber with ultraviolet light, the plasmatic substance and the water or a solute contained in the aqueous solution being reacted at a phases interface in the reaction chamber, wherein a diffuser fan is disposed in the reaction chamber.

In the phases interface reactor according to the invention, the water/aqueous solution supply means may be nebulization means that is configured for generating a mist of water or a mist of aqueous solution in the reaction chamber in a humidity-controllable manner. That is, the phases interface reactor may comprise a reaction chamber, plasma supply means that supplies a plasmatic substance into the reaction chamber, the nebulization means, and the ultraviolet irradiation means, and the plasmatic substance and the mist of water or a solute contained in the mist of aqueous solution may be reacted at a phases interface in the reaction chamber.

In the phases interface reactor according to the invention, a plasmatic substance and water or a solute contained in an aqueous solution are reacted at a phases interface between a plasma phase and a liquid phase in contact with the plasma phase. This allows the reaction to take place efficiently.

In the phases interface reactor according to the second invention, it is preferable that the nebulization means be a heater for heating the water or the aqueous solution and that the heater be disposed in the reaction chamber. With the use of a heater for heating water or the like to generate a mist, the humidity and the size of mist particles can easily be controlled well. In addition, the use of the heater can increase the temperature in the reaction chamber to increase the amount of saturated water vapor and thus increase the amount of water vapor in the reaction chamber.

The method for producing a secondary reaction product as described herein adapted for the above object comprises reacting a reaction product produced by the above phases interface reaction with another substance to produce a secondary reaction product.

In the method for producing a secondary reaction product as described herein, the reaction product produced by the phases interface reaction may comprise active oxygen or hydroxy radical.

In the phases interface reactor according to the invention, a reaction product produced by a phases interface reaction between the plasmatic substance and the water or a solute contained in the aqueous solution can be reacted with another substance inside or outside the reaction chamber to produce a secondary reaction product.

The use of the above method for producing a secondary reaction product or the use of the phases interface reactor for carrying out the method allows establishment of a new reaction method performed at normal temperature and pressure in the absence of a catalyst by making use of a reaction product obtained by a phases interface reaction. In particular, new organic synthesis or new metal surface treatment can be accomplished by reacting the above reaction product with another substance (an organic compound or metal, for example). When the reaction product comprises active oxygen or hydroxy radical, a reaction between the reaction product and an organic compound can result in oxidation of the organic compound, and a reaction between the reaction product and a metal can result in oxidation of the surface of the metal.

### Advantageous Effect of Invention

With the method for producing a reaction product using a phases interface reaction according to the first invention and the phases interface reactor according to the second invention, a plasmatic substance can be efficiently reacted with water or the like. With the method for producing a secondary reaction product as described herein, the reaction product obtained by the phases interface reaction can be used to accomplish new synthesis or new surface treatment. Thus, the present invention can increase, for example, the productivity of various types of chemical synthesis and can be used in various fields such as material processing (ex. anticorrosion processing or modification), sanitation technology (ex. sterilization, dust elimination or virus elimination), and renewable energy industry (ex. hydrogen purification).

### Brief Description of Drawings

[Figure 1] Figure 1 is a schematic diagram showing a phases interface reactor according to an embodiment of the present invention.
[Figure 2] Figure 2 shows a variant of the phases interface reactor of Figure 1.
[Figure 3] Figure 3 schematically shows typical modes of the phases interface reaction between a plasmatic substance and water or an aqueous solution.
[Figure 4] Figure 4(a) shows an ESR spectrum of DMPO-OH in a 0.3 M aqueous solution of DMPO (supplied ozone volume: 4 L/min × 10 min, ultraviolet irradiation time: 10 min), and Figure 4(b) shows an ESR spectrum of TPC-¹O₂ in a 0.5 M aqueous solution of TPC (supplied ozone volume: 4 L/min × 10 min, ultraviolet irradiation time: 10 min).
[Figure 5] Figure 5 is a graph showing the relationship between the ultraviolet irradiation time and the amount of produced TPC-¹O₂ (supplied ozone volume: 4 L/min × 2 min) .
[Figure 6] Figure 6 is a graph showing the relationship between the supplied ozone volume and the amount of produced TPC-¹O₂ (ozone supply rate: 4 L/min, ultraviolet irradiation time: 1 min).
[Figure 7] Figure 7 is a graph showing the relationship between the humidity and the amount of produced TPC-¹O₂.
[Figure 8] Figure 8 shows a comparison of the amount of produced ammonia among three systems, "N plasma phase/aqueous phase + UV irradiation", "N₂ gas phase/aqueous phase + UV irradiation", and "N plasma phase/aqueous phase".
[Figure 9] Figure 9 shows NMR spectra measured before and after treatment of indole with active oxygen formed by a phases interface reaction.
[Figure 10] Figure 10 shows an attenuated total reflection (ATR) FT-IR spectrum of the surface of a copper sheet treated with active oxygen formed by a phases interface reaction.

### Reference Signs List

10: Phases interface reactor, 11: Reaction chamber, 12: Plasma generator (an example of the plasma supply means), 13: Heater (an example of the water/aqueous solution supply means and an example of the nebulization means), 14: UV lamp (an example of the ultraviolet irradiation means), 15: Plasma inlet, 16: Inlet, 17: Outlet, 18: Pipe, 19: To-be-heated vessel (an example of the water/aqueous solution supply means), 20: Diffuser fan, 21, 22: Pipe, 30: Shower device (an example of the water/aqueous solution supply means), X: water or aqueous solution

### Description of Embodiments

Hereinafter, embodiments of the present invention will be described with reference to the accompanying drawings.

### [Phases interface reactor]

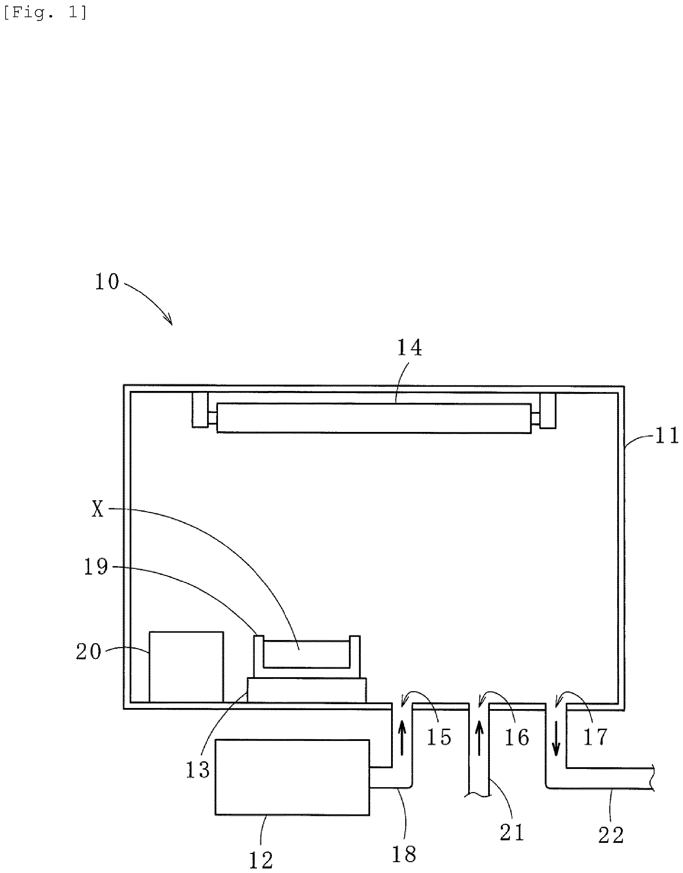

As shown in Figure 1, a phases interface reactor 10 according to an embodiment of the present invention mainly includes a reaction chamber 11, a plasma generator 12 as an example of the plasma supply means, a heater 13 as an example of the nebulization means, and a UV lamp 14 as an example of the ultraviolet irradiation means.

The reaction chamber 11 has an interior for reacting a plasmatic substance with a mist of water or the like. The reaction chamber 11 used may be a known chamber and may be a transparent chamber made of glass or the like or a non-transparent chamber made of ceramic or the like. The reaction chamber 11 is provided at its bottom with a plasma inlet 15 as well as with an inlet 16 and outlet 17 for another gas etc.

The plasma generator 12 generates plasma of a reactant and supplies the generated plasma (plasmatic substance) into the reaction chamber 11. Any known plasma generator exemplified by that makes use of discharge like arc discharge, makes use of high-frequency electromagnetic field or makes use of microwave, can be used as the plasma generator 12 as appropriate. The plasma generator 12 and the reaction chamber 11 are connected together via a pipe 18. Thus, the plasma generated by the plasma generator 12 is supplied through the pipe 18 and then the plasma inlet 15 to the reaction chamber 11.

The heater 13 is preferably disposed on the inner bottom surface of the reaction chamber 11. On the heater 13 there is placed a to-be-heated vessel 19 filled with water (or an aqueous solution) X. The to-be-heated vessel 19 is an example of the water/aqueous solution supply means that supplies water or an aqueous solution into the reaction chamber 11, and may not necessarily be heated. When the to-be-heated vessel 19 does not require heating, it can be simply referred to as "vessel". The heater 13 heats the to-be-heated vessel 19 and water X contained in the vessel. This heating evaporates the water X contained in the to-be-heated vessel 19, resulting in generation of a mist of water in the reaction chamber 11. The heater 13 used is not particularly limited and can be any heater capable of heating the to-be-heated vessel 19 and water contained in the to-be-heated vessel 19. For example, an electric heater, a gas heater, or a high-frequency induction heater can be employed.

The heater 13 generates a mist in a humidity-controllable manner in the reaction chamber 11. This humidity control can be accomplished by control of the heating temperature (the amount of heat applied to the water X). That is, the humidity can be increased by raising the heating temperature and thus increasing the amount of evaporation, and the humidity can be decreased by lowering the heating temperature and thus decreasing the amount of evaporation. Since the heater 13 and the to-be-heated vessel 19 are disposed in the reaction chamber 11, the heating by the heater 13 increases also the temperature in the reaction chamber 11. Thus, the heater 13 can increase the amount of saturated vapor in the reaction chamber 11 and hence increase the amount of water (water vapor and mist) containable in the atmosphere in the chamber. That is, the heater 13 acts to increase both the amount of water vapor and the amount of saturated water vapor, on the basis of which the humidity is determined. For example, when the heater 13 is an electric heater, the control of the heating temperature is accomplished by control of the electrical power.

The UV lamp 14 is disposed on the inner upper side of the reaction chamber 11. The UV lamp 14 irradiates the plasmatic substance supplied into the reaction chamber 11 from the plasma generator 12 with ultraviolet light. The UV lamp 14 also irradiates the mist of water and the water X contained in the to-be-heated vessel 19 with ultraviolet light. The wavelength of the ultraviolet light emitted by the UV lamp 14 is appropriately set depending on, for example, the type of the reactant. For example, when the reactant is oxygen (ozone), the wavelength can be 185 nm and 254 nm. The output power of the UV lamp 14 is not particularly limited and can be, for example, 0.1 to 100 W.

A diffuser fan 20 is further disposed in the reaction chamber 11. The diffuser fan 20 diffuses the plasmatic substance and the mist of water or the like over the interior of the reaction chamber 11. To the inlet 16 of the reaction chamber 11 there is connected a pipe 21, which allows a reactant gas or the like to be supplied from an unillustrated pump or the like. To the outlet 17 of the reaction chamber 11 there is connected a pipe 22, which allows the produced gas, unreacted gas and the like to be exhausted. The plasma inlet 15, inlet 16, and outlet 17 can be configured to be openable and closable.

In the reaction chamber 11 of the phases interface reactor 10, the plasmatic substance and the mist of water or the like react at a phases interface between the plasma phase and the liquid phase dispersed in the form of a mist over the plasma phase. Gaseous water (water vapor) resulting from evaporation can also participate in the reaction. This reaction will be described later for the method for using the phases interface reactor 10 and the method for producing a reaction product using a phases interface reaction.

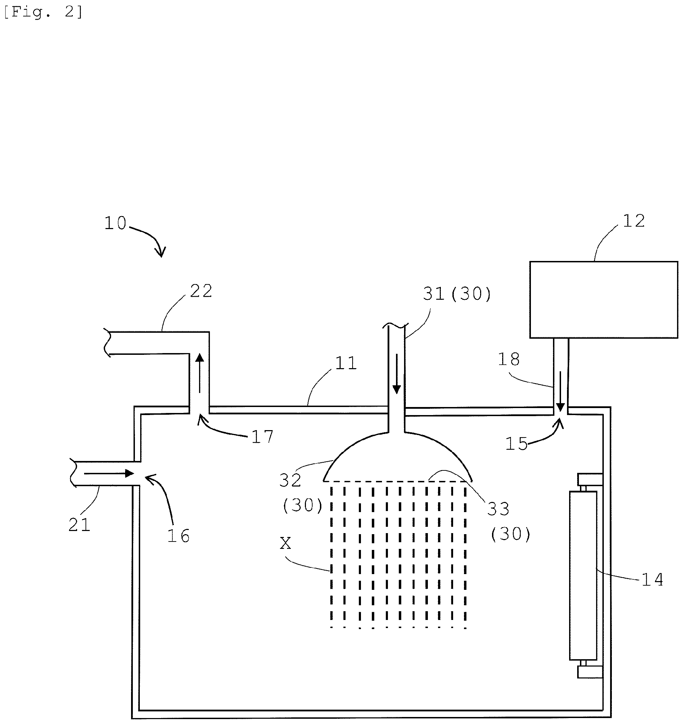

Figure 2 shows a variant of the phases interface reactor of Figure 1.

As shown in Figure 2, a phases interface reactor 10 mainly includes a reaction chamber 11, a plasma generator 12 as an example of the plasma supply means, a UV lamp 14 as an example of the ultraviolet irradiation means, and a shower device 30 as an example of the water/aqueous solution supply means. The phases interface reactor 10 of Figure 2 differs from the phases interface reactor 10 of Figure 1 by including the shower device 30 instead of the heater 13, to-be-heated vessel 19 and has the other components in common with the phases interface reactor 10 of Figure 1. The common components are as described above and will not be described again. The phases interface reactor 10 of Figure 2 includes the diffuser fan 20.

The shower device 30 includes a pipe 31 extending into the reaction chamber 11 and a shower head 32 provided at the termination of the pipe. The shower head 32 has as its lower surface a multiport face 33 having a large number of small ports. Water or an aqueous solution containing a solute is supplied into the reaction chamber 11 through the multiport face 33 of the shower head 32, and reacts with the plasmatic substance separately supplied into the reaction chamber 11 at the phases interface.

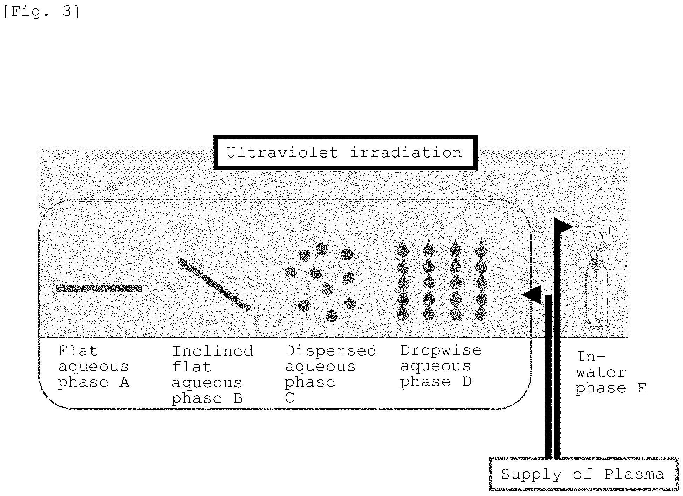

Figure 3 schematically shows typical modes of the phases interface reaction between the plasmatic substance and water or an aqueous solution ("water or an aqueous solution" will hereinafter be collectively referred to as "water"). The plasmatic substance may be any of various plasmas typified by nitrogen plasma.

The modes shown in Figure 3 are as follows.

### (A) Flat aqueous phase (a type of flat interface)

The plasmatic substance reacts at the surface of water contained in a vessel.

### (B) Inclined flat aqueous phase (a type of flat interface)

The plasmatic substance reacts at the surface of water flowing on an inclined face.

### (C) Dispersed aqueous phase

The plasmatic substance reacts at the surface of water vapor dispersed in the form of a mist over the interior of the chamber.

### (D) Dropwise aqueous phase

The plasmatic substance reacts at the surfaces of water droplets added into the chamber.

### (E) In-water phase

The plasmatic substance is bubbled into water filling the chamber or water in a water bath placed in the chamber and reacts with water at the surfaces of the bubbles.

The modes of the aqueous phase-plasma phases interface include the flat interfaces ((A) and (B)), the dispersed aqueous phase (C), the dropwise aqueous phase (D), and the in-water phase (E), as described above. Any of these phases interfaces can serve as a reaction site. The modes of the plasma-water phases interfaces (A), (B), (C), and (D) are suitable for increasing the efficiency of ultraviolet irradiation or of proton donation/acceptance at the aqueous interface to enhance the reaction efficiency.

The phases interface reactor 10 of Figure 1 forms an aqueous phase-plasma phases interface corresponding to (C) shown in Figure 3, and preferably includes the heater 13 as the nebulization means that generates a mist of water or a mist of aqueous solution in the reaction chamber 11 in a humidity-controllable manner. The heater 13 is not an essential component of the phases interface reactor 10, and the shower device 30 may be provided alone as in the phases interface reactor 10 of Figure 2. In this case, the mode of the aqueous phase-plasma phases interface corresponds to (D) in Figure 3.

The phases interface reactor 10 of Figure 1 may include the to-be-heated vessel 19 alone and cause the plasmatic substance (ex. ozone or nitrogen plasma) to react at the surface of water contained in the to-be-heated vessel 19. In this case, the mode of the aqueous phase-plasma phases interface corresponds to (A) in Figure 3. Alternatively, an inclined plate may be disposed in the reaction chamber 11 instead of disposing the to-be-heated vessel 19 inside the reaction chamber 11. Water may be pumped from the to-be-heated vessel 19 up to the upper portion of the inclined plate and moved downward along the surface of the inclined plate toward the to-be-heated vessel 19 to cause a reaction between the water flowing on the surface of the inclined plate and the plasmatic substance in contact with the surface of the water. In this case, the mode of the aqueous phase-plasma phases interface corresponds to (B) in Figure 3.

Alternatively, a bubbling device made of glass and filled with water may be disposed in the reaction chamber 11, and the plasmatic substance may be supplied and bubbled into the bubbling device to cause a reaction between the water and the plasmatic substance at the surfaces of the bubbles generated. In this case, the mode of the aqueous phase-plasma phases interface corresponds to (E) in Figure 3.

### [Method for producing reaction product using phases interface reaction]

The method for using the phases interface reactor 10 (or the method for producing a reaction product using a phases interface reaction) includes: a plasma supply step of supplying a plasmatic substance into the reaction chamber 11, the plasmatic substance being produced by supplying nitrogen gas into a plasma generator 12; a water/aqueous solution supply step of supplying water or an aqueous solution into the reaction chamber 11; and an ultraviolet irradiation step of irradiating the plasmatic substance in the reaction chamber 11 with ultraviolet light, wherein the humidity in the reaction chamber 11 is 40% or more and 70% or less during the ultraviolet irradiation step, the plasmatic substance and the water or a solute contained in the aqueous solution being reacted at a phases interface in the reaction chamber 11.

In the method for producing a reaction product using a phases interface reaction (this method may hereinafter be referred to as "reaction product producing method"), the water/aqueous solution supply step may be a nebulization step of generating a mist of water or a mist of aqueous solution in the reaction chamber 11, and the ultraviolet irradiation step may be a step of irradiating the plasmatic substance in the reaction chamber 11 with ultraviolet light, with the humidity in the reaction chamber 11 being 40% or more and 70% or less. The order of performing the plasma supply step, nebulization step, and ultraviolet irradiation step is not particularly limited as long as a phases interface reaction occurs. In general, it is preferable to allow all of these steps to take place simultaneously or carry out at least the nebulization step and ultraviolet irradiation step simultaneously.

### (Plasma supply step)

In the plasma supply step, the plasma generator 12 is operated to supply a plasmatic substance into the reaction chamber 11. Nitrogen gas (nitrogen molecules) is supplied to the plasma generator 12, so that nitrogen plasma (plasmatic nitrogen) in the form of a mixture of nitrogen atoms (N), nitrogen molecules, dissociated ions, electrons etc. is supplied to the reaction chamber 11.

The rate of supply of the plasmatic substance to the reaction chamber 11 is not particularly limited and is appropriately set depending on the apparatus size etc. The rate of supply can be, for example, about 0.1 L/min to 100 L/min.

### (Water/aqueous solution supply step)

In the water/aqueous solution supply step, water or an aqueous solution to be brought into contact with the plasmatic substance supplied from the plasma generator 12 is supplied into the reaction chamber 11. The supply method is not particularly limited, and various methods are available as described with reference to Figure 3. For example, when the water/aqueous solution supply step is a nebulization step of generating a mist of water or a mist of aqueous solution in the reaction chamber 11, the nebulization step is as described below.

### (Nebulization step)

In the nebulization step, the heater 13 is operated to generate a mist (a mist of water or a mist of aqueous solution) in the reaction chamber 11. That is, the heater 13 increases the temperature of water (or aqueous solution) X in the to-be-heated vessel 19, so that the water (or aqueous solution) X evaporates and then condenses into fine droplets dispersed in the atmosphere in the reaction chamber. The temperature of heating by the heater 13 (the temperature in the reaction chamber 11) is not particularly limited, and is preferably 30°C or higher and 50°C or lower and more preferably 35°C or higher and 45°C or lower. Heating in the above temperature range can generate a mist suitable for the phases interface reaction. Too high a temperature may cause an increase in the size of the mist particles and hence a decrease in the reaction efficiency or cause evaporation excessive relative to the increase in the amount of saturated water vapor and thus allow the humidity to reach 100%, leading to generation of a large amount of dew drops.

An aqueous solution of a solute can be heated instead of pure water to generate a mist of aqueous solution. The solute is not particularly limited, and may be any solute soluble in water and reactive with the plasma. For example, the solute may be an organic substance such as an alcohol or carboxylic acid or may be an inorganic substance such as ammonia or a metal salt. The solute may be either an electrolyte or a non-electrolyte. When the solute in the aqueous solution has a boiling point considerably different from that of water, a certain amount of the solute is incorporated in the mist, although the concentration of the solute in the mist is relatively low.

### (Ultraviolet irradiation step)

In the ultraviolet irradiation step, the plasmatic substance supplied from the plasma generator 12 and being present in the reaction chamber 11 is irradiated with ultraviolet light. The ultraviolet light is emitted from the UV lamp 14. This ultraviolet irradiation is performed with the humidity (relative humidity) in the reaction chamber 11 being 40% or more and 70% or less. If the humidity is 100%, the reaction efficiency will be low, for example, because the ultraviolet light is predominantly absorbed by a large amount of water molecules present in the reaction sites (ex. the atmosphere in and wall surface of the reaction chamber). Performing the ultraviolet irradiation under unsaturated water vapor conditions allows the reaction product to be collected basically in the form of a gas. The humidity in the reaction chamber 11 during the ultraviolet irradiation is 40% or more and 70% or less and preferably 45% or more and 65% or less. Too low a humidity causes a decrease in the amount of water dispersed in the reaction site (the atmosphere in the reaction chamber 11) and results in a reduction in the amount of the reaction product. Too high a humidity tends to increase the amount of ultraviolet light absorbed by water, and is likely to cause local formation of a large amount of dew drops even if the humidity is below 100%. The ultraviolet irradiation time is not particularly limited and is appropriately set depending on, for example, the type of the plasma and the amount of water. The irradiation time can be, for example, 0.1 minutes or more and 30 minutes or less.

The ultraviolet irradiation causes a reaction of the plasmatic substance with water (or a solute contained in an aqueous solution), preferably with a mist of water (or a solute contained in a mist of aqueous solution), at the phases interface in the reaction chamber 11. Since the plasmatic substance and water or a solute contained in an aqueous solution are reacted at the phases interface between the plasma phase and the liquid phase in contact with the plasma phase, a large area of contact between the two components is provided, which allows the reaction to take place efficiently.

Nitrogen atoms (plasma) react with water at the phases interface under the action of ultraviolet irradiation to produce ammonia etc. Ammonia is a component of the reaction product produced by the phases interface reaction. By supplying the generated ammonia into the plasma generator 12 (discharge device), the ammonia can be decomposed to obtain hydrogen molecules (and nitrogen molecules) (decomposition reaction step). In this way, hydrogen molecules can be obtained from nitrogen (air) and water as starting materials through production of ammonia.

The method for synthesizing ammonia using a phases interface reaction is a synthesis method that employs air or nitrogen and water as starting materials and makes use of a phenomenon where an atomic gas generated in plasma efficiently accepts dissociated protons by itself at the phases interface between the plasma and water (plasma-water phases interface). Passing nitrogen molecules through the discharge space of the plasma generator 12 causes conversion of nitrogen to plasma. Quickly forming a phases interface between the nitrogen plasma and water results in production of ammonia. The efficiency of ammonia synthesis can be increased by giving a reaction energy to the nitrogen plasma-water phases interface through irradiation with ultraviolet light.

Ammonia is a gas at normal pressure and has very high water solubility (NH₃ 702 g/H₂O 100 g at 20°C). The synthesized ammonia is thus dissolved in the aqueous phase (a portion of the synthesized ammonia may be present in the form of a gas in the atmosphere depending on the conditions of the reaction system). The synthesized ammonia can thus be collected easily as dissolved ammonia in the aqueous phase. Depending on the conditions of the reaction system, the synthesized ammonia can easily be collected as a gas, since the proportion of dissolved ammonia is significantly decreased at a high temperature (for example, the proportion of dissolved ammonia is 1/8 at 100°C).

The Haber-Bosch process, which has been traditionally well-known, synthesizes ammonia at high temperature and pressure in the presence of a catalyst, while the phases interface reaction described above can take place at normal temperature and pressure in the absence of a catalyst. It is thus possible to allow the synthesis reaction of ammonia to take place with a small input of energy, which is highly beneficial. This technique has many advantages in that: starting materials are available everywhere (transportation is unnecessary); the material cost is very low since ammonia can be synthesized using air and water as starting materials; carbon dioxide is not produced, which reduces the environmental load and eliminates the cost of transfer of carbon dioxide; the apparatus used is a simplified apparatus that operates at normal temperature and pressure; the reaction system is a low-energy reaction system (system of non-equilibrium chemical reaction at a phases interface); and the energy cost can be considerably reduced since production of hydrogen from a hydrocarbon fuel is unnecessary. In synthesis of ammonia from air and water, the presence of oxygen in air leads to a reaction between nitrogen plasma and oxygen plasma, thus producing a small amount of NO in the gas phase. However, NO is believed not to be mixed with ammonia contained in the liquid phase, since NO is never dissolved in the aqueous phase and can easily be exhausted in the form of a gas. The fact that NO has significantly lower water solubility than ammonia is one possible reason why ammonia can be produced inexpensively and safely.

The present invention is not limited to the above embodiments For example, in the nebulization step, a mist may be delivered into the reaction chamber 11 from the outside of the reaction chamber 11. The nebulization means used can be other than the heater 13 and may be a device based on ultrasonic waves (ultrasonic humidifier) or a spray that generates a mist in a physical manner. The ultraviolet irradiation means (UV lamp 14) can be disposed outside the reaction chamber 11 for irradiation with ultraviolet light from the outside of reaction chamber 11. Energy input means based on light or electromagnetic waves such as an excimer lamp (a lamp that emits vacuum ultraviolet light with a wavelength of 180 nm or less) may be used as the ultraviolet irradiation means instead of or in combination with the UV lamp 14.

### [Method for producing secondary reaction product]

The method for producing a secondary reaction product is a method in which a reaction product formed by the above phases interface reaction and another substance are reacted to produce the secondary reaction product. The phases interface reaction is performed between nitrogen plasma and water, the reaction product refers to ammonia. The phases interface reaction may be performed in any mode selected from the flat aqueous phase (A) (a type of flat interface), inclined flat aqueous phase (B) (a type of flat interface), dispersed aqueous phase (C), dropwise aqueous phase (D), and in-water phase (E) which have been previously described (see Figure 3). The other substance is not particularly limited and may be any substance reactive with the above reaction product. Examples of the other substance include organic compounds and inorganic compounds (including metals). When the reaction product is a substance such as active oxygen which has the nature of oxidizing the other substance, the other substance may be referred to as a to-be-oxygenized compound or to-be-oxygenized substance.

The reaction product (hydroxy radical, singlet oxygen, etc.) obtained by the phases interface reaction between oxygen plasma (not according with the invention) and water is an active substance having high electron-withdrawing ability. Thus, a reaction of this reaction product with an organic compound (ex. indole) allows the organic compound to be oxidized in a short time. The indole may be dispersed or dissolved in a solvent. For example, when the solvent used is water, it is preferable to prepare an aqueous solution containing 1 to 5 wt% of indole. When the solvent used is an organic solvent such as ethanol, it is preferable to prepare a solution containing 10 to 30 wt% of indole. The above content of indole is only illustrative, and a solution having a different indole content may be used. This reaction can be performed at normal temperature and pressure in the absence of a catalyst and is thus beneficial as an inexpensive technique for organic synthesis. The other substance to be reacted with the reaction product formed by the phases interface reaction may be other than indole, and examples of substances that can be used include skatole, phenylalanine, indoleacetate, hydrogen peroxide, aldehyde, alcohol, flavonoid, anthocyanine, flavone, quercetin, catechin, pyrrole, styrene, thiophene, benzaldehyde, and indene.

In addition, a reaction of the reaction product with a metal (ex. copper) allows the surface of the metal to be oxidized in a short time. This reaction can also be performed at normal temperature and pressure in the absence of a catalyst and is thus beneficial as an inexpensive technique for metal surface treatment. The metal may be other than copper, and examples of the metal that can be used include iron, nickel, and zinc.

The method for producing a secondary reaction product can be implemented inside or outside the phases interface reactor 10 described above. When the secondary reaction product is produced outside the phases interface reactor 10, it is recommended to prepare another reactor connected to the phases interface reactor 10 via a pipe, introduce the reaction product obtained by the phases interface reaction method into the other reactor through the pipe, and bring the reaction product into contact with another substance (ex. an organic compound or metal) placed inside the other reactor.

When the secondary reaction product is produced inside the phases interface reactor 10, it is recommended to bring the reaction product generated in the reactor 10 into contact with an organic compound by placing, in the reaction chamber 11 of the reactor 10, a vessel such as a petri dish filled with the organic compound or with a liquid containing the organic compound mixed with a certain solvent (ex. water). When a vessel such as a petri dish filled with water containing a dissolved or dispersed organic compound is used, it is preferable that a vessel different from the petri dish be separately prepared as the to-be-heated vessel 19 filled with water to be reacted with the plasmatic substance. Alternatively, a petri dish filled with water containing a dispersed or dissolved organic compound may be placed alone in the reaction chamber 11 so that the reaction product obtained by reaction between the water in the petri dish and the plasmatic substance is reacted with the organic compound in the petri dish to produce the secondary reaction product. When the reaction product and a metal are reacted, it is recommended to place the metal in the reaction chamber 11 of the reactor 10 and bring the reaction product generated in the reaction chamber 11 into contact with the metal. The form of the metal is not limited, and the metal may be in the form of a sheet, powder, or the like. When a sheet-shaped metal is to be surface-treated, it is recommended to place the sheet-shaped metal in the reaction chamber 11. When a metal powder is to be surface-treated, it is recommended to shake or stir the powder under explosion-proof conditions at a predetermined place in the reaction chamber 11 and bring the reaction product and the metal powder into contact at the predetermined place.

As described above, the phases interface reactor 10 can be used to produce a secondary reaction product by reacting a reaction product, formed by a phases interface reaction between a plasmatic substance and water or a solute in an aqueous solution, with another substance (ex. an organic compound or metal) inside or outside the reaction chamber 11. This allows establishment of, for example, a new organic synthesis method or a new metal surface treatment method.

### Examples

Hereinafter, the present invention will be described in more detail with reference to examples. The present invention is not limited to the examples given below.

### <Example 1> (not according with the invention) Production of hydroxy radical and singlet oxygen

A phases interface reactor having the configuration shown in Figure 1 was prepared. Two UV lamps having a wavelength of 185 nm and an output power of 0.16 W and two UV lamps having a wavelength of 254 nm and an output power of 1.6 W were used. A sample stage for placement of a hole slide glass plate described later was placed in the reaction chamber. The plasma generator (ozone generator) was allowed to discharge at 6 kV to supply oxygen plasma (ozone) into the reaction chamber, and the heater was operated to generate a mist (a mist of water) in the reaction chamber. The heating by the heater was performed so that the temperature in the reaction chamber reached 40°C. The humidity in the reaction chamber was 50%. In this state, ultraviolet light was emitted from the UV lamps.

Hydroxy radical and singlet oxygen as the resulting products were detected by ESR-spin trapping. A 0.3 M aqueous solution (500 µL) of 5,5-dimethyl-1-pyrroline N-oxide (DMPO; produced by LABOTEC Co., Ltd.) and a 0.5 M aqueous solution (500 µL) of 2,2,5,5-tetramethyl-3-pyrroline-3-carboxamide (TPC; produced by Sigma-Aldrich Co., LLC.) were used as spin trapping agents, and a drop of each of these solutions was placed on a hole slide glass plate, which was set on the sample stage in the reaction chamber. DMPO traps the hydroxy radical to form DMPO-OH as a spin adduct, while TPC traps the singlet oxygen to form TPC-¹O₂ as a spin adduct. The ESR was measured at room temperature using JFS-FA100 produced by JEOL Ltd.

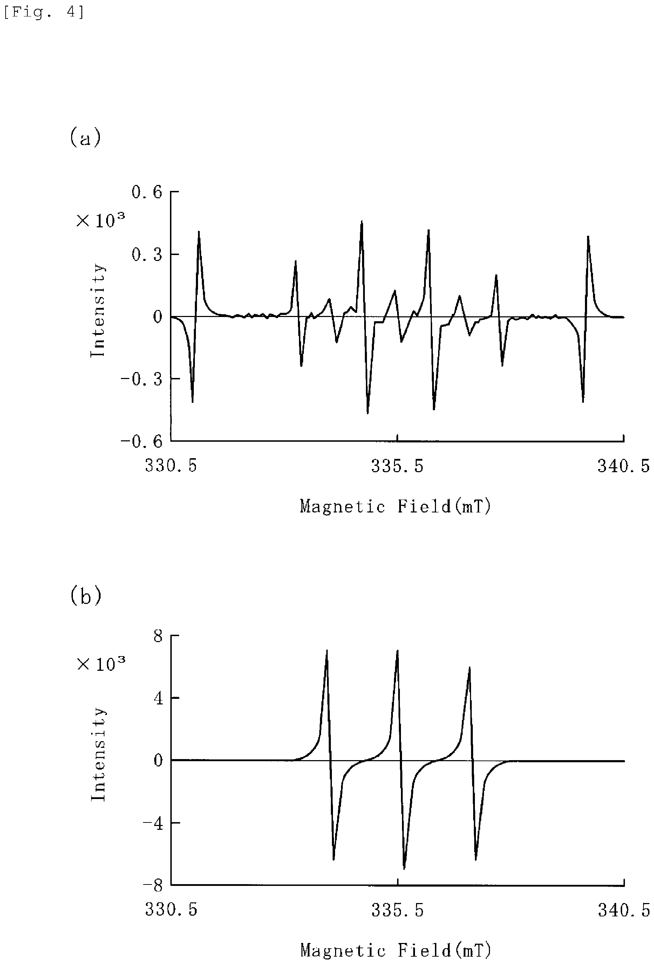

Supply of oxygen plasma (ozone) at a rate of 4 L/min and ultraviolet irradiation were performed for 10 minutes, after which the ESR spectra were measured for the spin trapping agents (spin adducts). The spectra are shown in Figure 4(a) and Figure 4(b), respectively. The concentrations of DMPO-OH and TPC-¹O₂ as determined from these spectra were high and 9 × 10⁻³ mM and 15 mM, respectively.

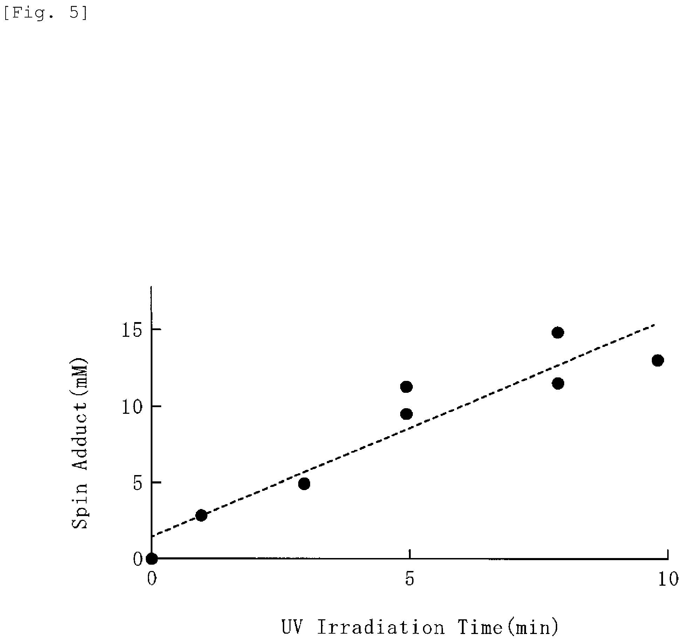

Next, the same reaction was performed by varying the ultraviolet irradiation time (the ozone supply was done at a rate of 4 L/min for 2 minutes). After that, the concentration of the spin adduct (TPC-¹O₂) was measured in the same manner as above. The results are shown in Figure 5. In Figure 5, the abscissa represents the ultraviolet irradiation time (UV Irradiation Time (min)), and the ordinate represents the concentration of the spin adduct (Spin Adduct (TPC-¹O₂) (mM)). It can be understood that the longer the ultraviolet irradiation time is, the larger the amount of the spin adduct (singlet oxygen) produced is, and thus that the ultraviolet irradiation promotes the production of singlet oxygen.

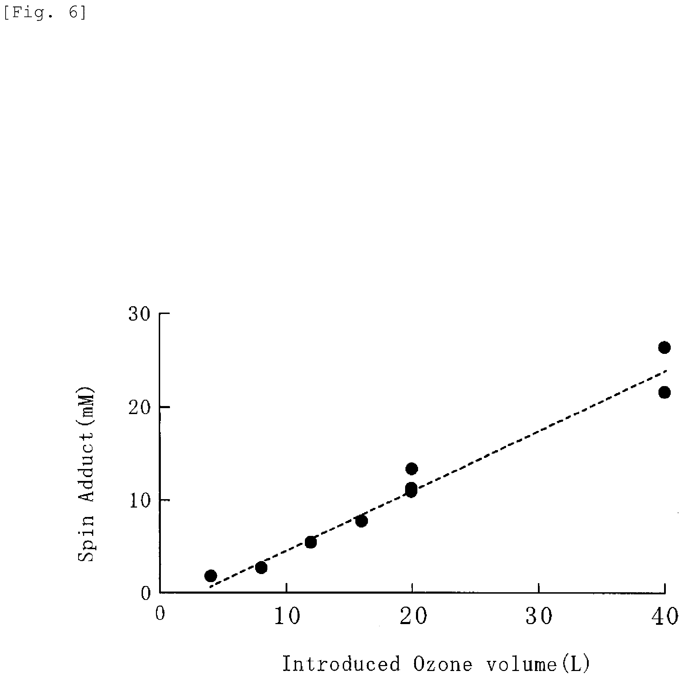

The same reaction was performed by varying the volume of supplied oxygen plasma (ozone) (the rate of ozone supply was 4 L/min and the ultraviolet irradiation time was 1 minute). After that, the concentration of the spin adduct (TPC-¹O₂) was measured in the same manner as above. The results are shown in Figure 6. In Figure 6, the abscissa represents the volume of supplied (introduced) ozone (Introduced Ozone volume (L)) and the ordinate represents the concentration of the spin adduct (Spin Adduct (TPC-¹O₂) (mM)). It can be understood that the larger the volume of supplied oxygen is, the larger the amount of produced singlet oxygen is (there is a substantially proportional relationship). This is presumably because the chain reaction represented by above formulae (1) to (4) takes place at the reaction site.

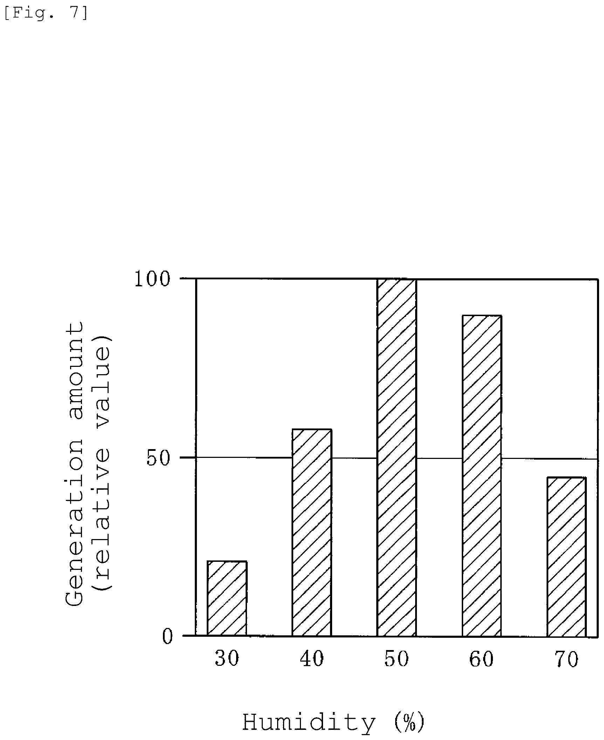

Next, the same reaction was performed by varying the humidity (relative humidity) in the reaction chamber. After that, the concentration (generation amount) of the spin adduct (TPC-¹O₂) was measured in the same manner as above. The results are shown in Figure 7 in the form of relative values determined by defining the generation amount obtained at a humidity of 50% as 100 (%). It can be understood that the singlet oxygen was produced at a high concentration when the humidity was in the range of 40 to 70%, in particular when the humidity was 50% or 60%. In this example, the water (or aqueous solution) in the reaction atmosphere (the space in the reaction chamber) was present both in the form of a gas (water vapor) and in the form of a dispersed liquid (mist). Thus, the value of the humidity can be used as a rough measure of the amount of water present in the atmosphere, although the value cannot be considered to exactly represent the amount of water present in the atmosphere.

### <Example 2> (according with the invention) Synthesis of ammonia

As in Example 1, a phases interface reactor having the configuration shown in Figure 1 was prepared. UV lamps identical to those in Example 1 were used. A nitrogen gas (purity: 99.99% or more) was supplied into the plasma generator (for nitrogen plasma generation), which was allowed to discharge at a voltage of 6 kV to supply nitrogen plasma into the reaction chamber. Ultrapure water in an amount of 20 ml was put in a petri dish with a diameter of 13 cm (aqueous phase surface area: 132 cm²) and heated by the heater to generate a mist (a mist of water) in the reaction chamber. The heating by the heater was performed so that the temperature in the reaction chamber reached 40°C. The humidity in the reaction chamber was 50%. In this state, ultraviolet light was emitted from the UV lamps to the surface of the aqueous phase for 10 minutes. This system will be referred to as "N plasma phase/aqueous phase + UV irradiation". The aqueous phase in the petri dish was colored by "indophenol blue coloring technique", then its absorbance was measured, and ammonia produced in the aqueous phase was precisely quantified by a standard calibration method.

For comparison, the amount of produced ammonia was examined for two other systems, one of which was "N₂ gas phase/aqueous phase + UV irradiation" differing from the above "N plasma phase/aqueous phase + UV irradiation" by including supply of nitrogen gas instead of supply of nitrogen plasma, and the other of which was "N plasma phase/aqueous phase" differing from the above "N plasma phase/aqueous phase + UV irradiation" by not including ultraviolet irradiation.

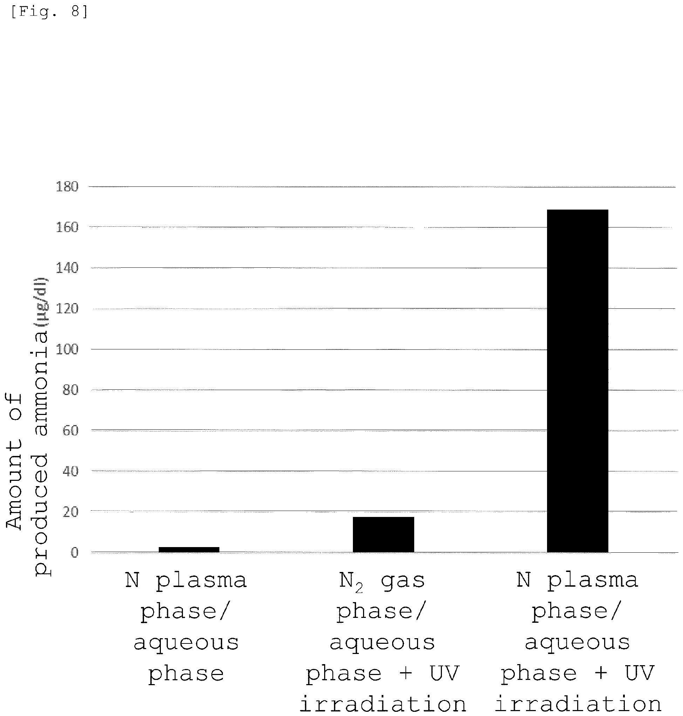

Figure 8 shows a comparison of the amount of produced ammonia among the three systems, "N plasma phase/aqueous phase + UV irradiation", "N₂ gas phase/aqueous phase + UV irradiation", and "N plasma phase/aqueous phase". Figure 8 clearly confirms that a considerably larger amount of ammonia was produced in "N plasma phase/aqueous phase + UV irradiation" than in the two other systems. This result demonstrates the superiority in ammonia synthesis of the method in which nitrogen gas is converted to plasma, and the plasma in contact with an aqueous phase is irradiated with UV. The energy consumed for synthesis of 170 µg of ammonia as represented by the rightmost bar in Figure 8 included only electricity used in silent discharge for nitrogen plasma generation and in ultraviolet irradiation of the reaction interface. The amount of the electricity was very small and specifically not more than 5 Wh.

### <Example 3> (not according with the invention) Oxidation of organic compound

As in Example 1, a phases interface reactor having the configuration shown in Figure 1 was prepared. An aqueous indole solution (aqueous solution containing 3 wt% of indole) in an amount of 20 ml was poured in a petri dish with a diameter of 13 cm (aqueous phase surface area: 132 cm²), and the petri dish was placed in the phases interface reactor. The petri dish used in this experiment was a vessel different from the to-be-heated vessel to be heated by the heater. UV lamps identical to those in Example 1 were used. The rate of supply of oxygen plasma (ozone) was 4 L/min as in Example 1. The oxygen plasma (ozone) was generated by a silent discharger at a voltage of 6 kV. A mist (a mist of water) was generated in the reaction chamber by means of the heater. The heating by the heater was performed so that the temperature in the reaction chamber reached 40°C. The humidity in the reaction chamber was 50%. In this state, UV irradiation was performed using the UV lamps for 10 minutes. The aqueous indole solution in the petri dish was continuously stirred with a stirrer.

The generation of oxygen plasma took place in a mode corresponding to the dispersed aqueous phase (C) shown in Figure 3. The site of reaction of indole is expressed as "oxygen plasma phase/dispersed aqueous phase/indole dissolved in aqueous solvent". In the mode corresponding to the dispersed aqueous phase (C), hydroxy radical and active oxygen (which will collectively be referred to as active oxygen hereinafter) generated at the interface between the oxygen plasma phase and the aqueous phase oxidize indole in the aqueous indole solution. That is, in the phases interface reactor there occur two reactions, one of which is the reaction of production of active oxygen and the other of which is the reaction between the produced active oxygen and indole. Conversion of indole caused by the reaction between active oxygen and indole was identified on the basis of a change in nuclear magnetic resonance (NMR) spectrum.

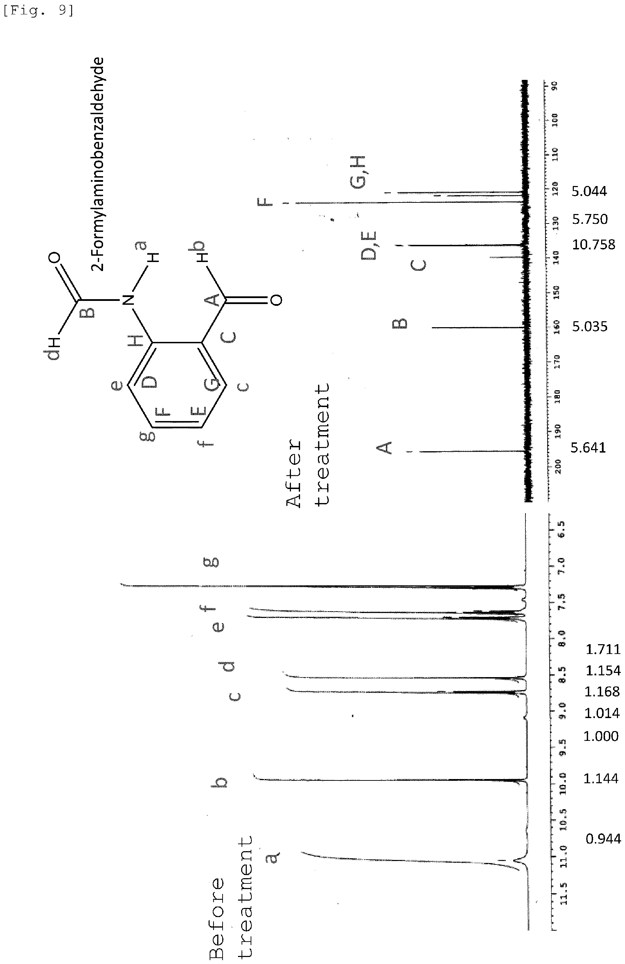

Figure 9 shows NMR spectra measured before and after treatment of indole with active oxygen produced by the phases interface reaction.

As shown in Figure 9, a NMR spectrum characteristic of indole was observed before treatment with active oxygen. After treatment with active oxygen, a NMR spectrum characteristic of 2-Formylaminobenzaldehyde was observed. This result leads to the inference that indole was oxidized by active oxygen and converted to 2-Formylaminobenzaldehyde by addition of oxygen or by ring opening. The reaction method using active oxygen produced by the phases interface reaction allows oxidation of organic compounds at normal temperature and pressure in the absence of a catalyst, and thus has a potential as a new technique for organic synthesis.

### <Example 4> (not according with the invention) Metal surface oxidation treatment

As in Example 1, a phases interface reactor having the configuration shown in Figure 1 was prepared. A 5-mm-wide, 20-mm-long, 0.5-mm-thick pure copper sheet (hereinafter referred to as "copper sheet"), which was polished, acid-treated, and cleaned by degreasing using an organic solvent beforehand, was placed in the phases interface reactor. UV lamps identical to those in Example 1 were used. The rate of supply of oxygen plasma (ozone) was 4 L/min as in Example 1. The oxygen plasma (ozone) was generated by a silent discharger at a voltage of 6 kV. A mist (a mist of water) was generated in the reaction chamber by means of the heater. The heating by the heater was performed so that the temperature in the reaction chamber reached 40°C. The humidity in the reaction chamber was 50%. In this state, UV irradiation was performed using the UV lamps for 5 minutes.

The generation of oxygen plasma took place in a mode corresponding to the dispersed aqueous phase (C) shown in Figure 3. The site of reaction of the copper sheet is expressed as "oxygen plasma phase/dispersed aqueous phase/copper sheet". In the mode corresponding to the dispersed aqueous phase (C), active oxygen oxidizes the surface of the copper sheet. That is, in the phases interface reactor there occur two reactions, one of which is the reaction of production of active oxygen and the other of which is the reaction between the produced active oxygen and copper. The reaction between active oxygen and copper was examined by infrared spectroscopy (attenuated total reflection (ATR) spectroscopy).

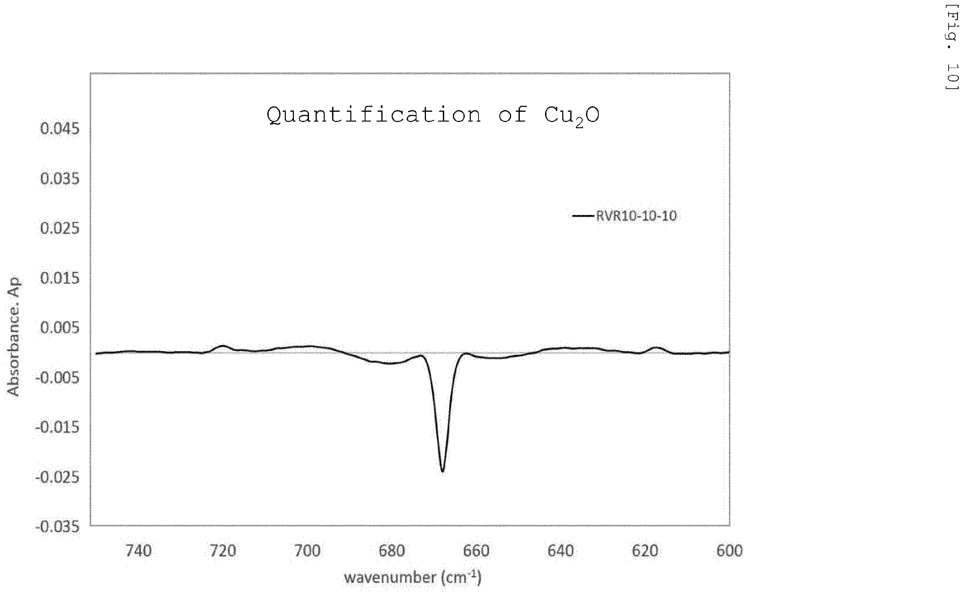

Figure 10 shows an attenuated total reflection (ATR) FT-IR spectrum of the surface of the copper sheet treated with active oxygen formed by the phases interface reaction.

In the spectrum shown in Figure 10, an infrared absorption peak characteristic of a Cu₂O layer is observed. This result indicates that a Cu₂O layer(copper suboxide layer) was formed in the surface of the copper sheet. Active oxygen and radicals have high electron-withdrawing ability and are thus capable of forming an oxide coating (suboxide film or oxide film) on a metal surface in a short time. A suboxide film or oxide film quickly formed is dense and thus capable of protecting the metal lying inwardly of the film from corrosion. In addition, a suboxide layer has an electrical rectification effect and thus contributes to inexpensive construction of a semiconductor material.

## Claims

1. A method for producing a reaction product including ammonia using a phases interface reaction, comprising:
a plasma supply step of supplying a plasmatic substance into a reaction chamber (11), the plasmatic substance being produced by supplying nitrogen gas into a plasma generator (12) ;
a water/aqueous solution supply step of supplying water or an aqueous solution into the reaction chamber (11); and
an ultraviolet irradiation step of irradiating the plasmatic substance in the reaction chamber (11) with ultraviolet light, wherein the humidity in the reaction chamber (11) is 40% or more and 70% or less during the ultraviolet irradiation step,
the plasmatic substance and the water or a solute contained in the aqueous solution being reacted at a phases interface in the reaction chamber (11).

2. The method for producing a reaction product including ammonia using a phases interface reaction of claim 1,
wherein
the water/aqueous solution supply step is a nebulization step of generating a mist of water or a mist of aqueous solution in the reaction chamber (11), and
the ultraviolet irradiation step is a step of irradiating the plasmatic substance in the reaction chamber (11) with ultraviolet light%.

3. The method for producing a reaction product including ammonia using a phases interface reaction of claim 2, wherein the nebulization step is accomplished by heating the water or the aqueous solution in the reaction chamber (11).

4. The method for producing a reaction product including ammonia using a phases interface reaction of any one of claims 1 to 3, wherein the ultraviolet irradiation step is the step of irradiating also the water or the aqueous solution with ultraviolet light.

5. The method for producing a reaction product including ammonia using a phases interface reaction of claim 4, wherein the method further comprising a decomposition reaction step of decomposing ammonia produced by the reaction at the phases interface.

6. A phases interface reactor (10) comprising:
a reaction chamber (11);
plasma supply means (12) configured for supplying a plasmatic substance into the reaction chamber (11);
water/aqueous solution supply means (13, 19, 30) configured for supplying water or an aqueous solution into the reaction chamber (11); and
ultraviolet irradiation means (14) configured for irradiating the plasmatic substance in the reaction chamber (11) with ultraviolet light,
the plasmatic substance and the water or a solute contained in the aqueous solution being reacted at a phases interface in the reaction chamber (11),
wherein a diffuser fan (20) is disposed in the reaction chamber (11).

7. The phases interface reactor (10) of claim 6, wherein
the water/aqueous solution supply means (13, 19, 30) is nebulization means (30) configured for generating a mist of water or a mist of aqueous solution in the reaction chamber (11) in a humidity-controllable manner, and
the plasmatic substance and the mist of water or the solute contained in the mist of aqueous solution are reacted at the phases interface in the reaction chamber (11).

8. The phases interface reactor (10) of claim 6 or 7, wherein the ultraviolet irradiation means (14) is also configured for irradiating the water or the aqueous solution with ultraviolet light.

9. The phases interface reactor (10) of any one of claims 6 to 8, wherein the reaction product produced by the phases interface reaction between the plasmatic substance and the water or the solute contained in the aqueous solution is reacted with another substance inside or outside the reaction chamber (11) to produce a secondary reaction product.

10. The phases interface reactor (10) of any one of claims 6 to 9, wherein the ultraviolet irradiation means (14) is a UV lamp (14).

## Patentansprüche

1. Verfahren zum Herstellen eines Reaktionsprodukts einschließlich Ammoniak unter Verwendung einer Phasenschnittstellenreaktion, aufweisend:
einen Plasmaspeiseschritt zum Einspeisen einer plasmatischen Substanz in eine Reaktionskammer (11), wobei die plasmatische Substanz durch Einspeisen von Stickstoffgas in einen Plasmagenerator (12) hergestellt wird;
einen Wasser-/wässrige Lösung-Speiseschritt zum Einspeisen von Wasser oder einer wässrigen Lösung in die Reaktionskammer (11); und
einen ultravioletten Bestrahlungsschritt zum Bestrahlen der plasmatischen Substanz in der Reaktionskammer (11) mit ultraviolettem Licht, wobei die Luftfeuchtigkeit in der Reaktionskammer (11) 40 % oder mehr und 70 % oder weniger während des ultravioletten Bestrahlungsschritts beträgt,
wobei die plasmatische Substanz und das Wasser oder ein in der wässrigen Lösung enthaltener gelöster Stoff an einer Phasenschnittstelle in der Reaktionskammer (11) zur Reaktion gebracht wird.

2. Verfahren zum Herstellen eines Reaktionsprodukts einschließlich Ammoniak unter Verwendung einer Phasenschnittstellenreaktion nach Anspruch 1, wobei der Wasser-/wässrige Lösung-Speiseschritt ein Zerstäubungsschritt zum Erzeugen eines Nebels aus Wasser oder eines Nebels aus wässriger Lösung in der Reaktionskammer (11) ist,
und
der ultraviolette Bestrahlungsschritt ein Schritt zum Bestrahlen der plasmatischen Substanz in der Reaktionskammer (11) mit ultraviolettem Licht ist.

3. Verfahren zum Herstellen eines Reaktionsprodukts einschließlich Ammoniak unter Verwendung einer Phasenschnittstellenreaktion nach Anspruch 2, wobei der Zerstäubungsschritt durch Erwärmen des Wassers oder der wässrigen Lösung in der Reaktionskammer (11) erreicht wird.

4. Verfahren zum Herstellen eines Reaktionsprodukts einschließlich Ammoniak unter Verwendung einer Phasenschnittstellenreaktion nach einem der Ansprüche 1 bis 3, wobei der ultraviolette Bestrahlungsschritt der Schritt zum Bestrahlen auch des Wassers oder der wässrigen Lösung mit ultraviolettem Licht ist.

5. Verfahren zum Herstellen eines Reaktionsprodukts einschließlich Ammoniak unter Verwendung einer Phasenschnittstellenreaktion nach Anspruch 4, wobei das Verfahren ferner einen Abbaureaktionsschritt zum Abbauen von Ammoniak aufweist, welches durch die Reaktion an der Phasenschnittstelle erzeugt wird.

6. Phasenschnittstellenreaktor (10), aufweisend:
eine Reaktionskammer (11);
ein Plasmaspeisemittel (12), welches zum Einspeisen einer plasmatischen Substanz in die Reaktionskammer (11) ausgelegt ist;
ein Wasser-/wässrige Lösung-Speisemittel (13, 19, 30), welches zum Einspeisen von Wasser oder einer wässrigen Lösung in die Reaktionskammer (11) ausgelegt ist; und
ein ultraviolettes Bestrahlungsmittel, welches zum Bestrahlen der plasmatischen Substanz in der Reaktionskammer (11) mit ultraviolettem Licht ausgelegt ist,
wobei die plasmatische Substanz und das Wasser oder ein in der wässrigen Lösung enthaltener gelöster Stoff an einer Phasenschnittstelle in der Reaktionskammer (11) zur Reaktion gebracht wird,
wobei ein Diffusorgebläse (20) in der Reaktionskammer (11) angeordnet ist.

7. Phasenschnittstellenreaktor (10) nach Anspruch 6, wobei das Wasser-/wässrige Lösung-Speisemittel (13, 19, 30) ein Zerstäubungsmittel (30) ist, welches zum Erzeugen eines Nebels aus Wasser oder eines Nebels aus wässriger Lösung in der Reaktionskammer (11) in einer Luftfeuchtigkeit steuerbaren Weise ausgelegt ist, und
die plasmatische Substanz und der Nebel aus Wasser oder der in dem Nebel aus wässriger Lösung enthaltene gelöste Stoff an der Phasenschnittstelle in der Reaktionskammer (11) zur Reaktion gebracht wird.

8. Phasenschnittstellenreaktor (10) nach Anspruch 6 oder 7, wobei das ultraviolette Bestrahlungsmittel (14) auch zum Bestrahlen des Wassers oder der wässrigen Lösung mit ultraviolettem Licht ausgelegt ist.

9. Phasenschnittstellenreaktor (10) nach einem der Ansprüche 6 bis 8, wobei das Reaktionsprodukt, welches durch die Phasenschnittstellenreaktion zwischen der plasmatischen Substanz und dem Wasser oder dem in der wässrigen Lösung enthaltenen gelösten Stoff hergestellt wird, mit einer anderen Substanz innerhalb oder außerhalb der Reaktionskammer (11) zur Reaktion gebracht wird, um ein sekundäres Reaktionsprodukt herzustellen.

10. Phasenschnittstellenreaktor (10) nach einem der Ansprüche 6 bis 9, wobei das ultraviolette Bestrahlungsmittel (14) eine UV-Lampe (14) ist.

## Revendications

1. Un procédé pour la production d'un produit de réaction comprenant de l'ammoniaque utilisant une réaction à l'interface de phases, comprenant :
une étape d'incorporation de plasma consistant à incorporer une substance plasmatique dans une chambre de réaction (11), la substance plasmatique étant produite par l'incorporation d'azote gazeux dans un générateur de plasma (12) ;
une étape d'incorporation d'eau / de solution aqueuse consistant à incorporer de l'eau ou une solution aqueuse dans la chambre de réaction (11) ; et
une étape d'irradiation ultraviolette consistant à irradier la substance plasmatique dans la chambre de réaction (11) avec une lumière ultraviolette, étant précisé que l'humidité dans la chambre de réaction (11) est de 40% ou plus et de 70% ou moins pendant l'étape d'irradiation,
la substance plasmatique et l'eau ou un soluté contenu dans la solution aqueuse étant mis à réagir à une interface de phases dans la chambre de réaction (11).

2. Le procédé pour la production d'un produit de réaction comprenant de l'ammoniaque utilisant une réaction à l'interface de phases selon la Revendication 1, étant précisé que
l'étape d'incorporation de l'eau / de la solution aqueuse est une étape de nébulisation consistant à générer un brouillard d'eau ou un brouillard de solution aqueuse dans la chambre de réaction (11), et
l'étape d'irradiation ultraviolette est une étape consistant à irradier la substance plasmatique dans la chambre de réaction (11) avec une lumière ultraviolette.

3. Le procédé pour la production d'un produit de réaction comprenant de l'ammoniaque utilisant une réaction à l'interface de phases selon la Revendication 2, étant précisé que l'étape de nébulisation est réalisée en chauffant l'eau ou la solution aqueuse dans la chambre de réaction (11).

4. Le procédé pour la production d'un produit de réaction comprenant de l'ammoniaque utilisant une réaction à l'interface de phases selon l'une quelconque des Revendications 1 à 3, étant précisé que l'étape d'irradiation ultraviolette est une étape consistant à irradier également l'eau ou la solution aqueuse avec une lumière ultraviolette.

5. Le procédé pour la production d'un produit de réaction comprenant de l'ammoniaque utilisant une réaction à l'interface de phases selon la Revendication 4, étant précisé que le procédé comprend en outre une étape de réaction de décomposition **caractérisée par** la décomposition de l'ammoniaque produite par la réaction à l'interface de phases.

6. Un réacteur d'interface de phases (10) comprenant :
une chambre de réaction (11) ;
un dispositif d'alimentation en plasma (12) configuré pour alimenter la chambre de réaction (11) en substance plasmatique ;
un dispositif d'alimentation en eau / solution aqueuse (13, 19, 30) configuré pour alimenter la chambre de réaction (11) en eau / solution aqueuse ; et
un dispositif d'irradiation ultraviolette (14) configuré pour irradier la substance plasmatique dans la chambre de réaction (11) avec une lumière ultraviolette,
la solution plasmatique et l'eau ou un soluté contenu dans la solution aqueuse étant mis à réagir à une interface de phases dans la chambre de réaction,
étant précisé qu'un ventilateur de diffusion (20) est prévu dans la chambre de réaction (11).

7. Le réacteur d'interface de phases (10) de la Revendication 6, étant précisé que le dispositif d'alimentation en eau / solution aqueuse (13, 19, 30) est un dispositif de nébulisation (30) configuré pour générer un brouillard d'eau ou un brouillard de solution aqueuse dans la chambre de réaction (11) d'une manière permettant de contrôler l'humidité, et
la substance plasmatique et le brouillard d'eau ou le soluté contenu dans le brouillard de solution aqueuse sont mis à réagir à l'interface de phases dans la chambre de réaction (11).

8. Le réacteur d'interface de phases (10) de la Revendication 6 ou 7, étant précisé que le dispositif d'irradiation ultraviolette (14) est également configuré pour irradier l'eau ou la solution aqueuse avec la lumière ultraviolette.

9. Le réacteur d'interface de phases (10) de l'une quelconque des Revendications 6 à 8, étant précisé que le produit de la réaction produit par la réaction à l'interface des phases entre la substance plasmatique et l'eau ou le soluté contenu dans la solution aqueuse est mis à réagir avec une autre substance à l'intérieur ou à l'extérieur de la chambre de réaction (11) afin de produire un produit de réaction secondaire.

10. Le réacteur d'interface de phases (10) de l'une quelconque des Revendications 6 à 9, étant précisé que le dispositif d'irradiation ultraviolette (14) est une lampe à UV (14).
